# EUROPEAN PATENT SPECIFICATION

(11) **EP 4 218 564 B1**
(45) Date of publication and mention of the grant of the patent: **31.12.2025**
(21) Application number: 22213513.9
(22) Date of filing: 16.09.2019
(51) Int. Cl.: A61B 5/11, A61B 5/00, A61B 5/107

(54) **SENSOR AND METHOD FOR MEASURING MOVEMENT OF BREAST TISSUE DURING ACTIVITY**
SENSOR UND VERFAHREN ZUR MESSUNG DER BEWEGUNG VON BRUSTGEWEBE WÄHREND DER AKTIVITÄT
CAPTEUR ET PROCÉDÉ DE MESURE DU MOUVEMENT DU TISSU MAMMAIRE PENDANT L'ACTIVITÉ

(30) Priority: 20.09.2018 US 201862734217 P
(43) Date of publication of application: 02.08.2023
(62) Divisional of application: 19863650.8
(73) Proprietor: Lululemon Athletica Canada Inc., Vancouver British Columbia V6J 1C7 (CA)
(72) Inventor: Santry, Joseph John, Squamish (CA); Murnaghan, Chantelle Dawn, Vancouver (CA); Buckeridge, Erica Margaret, Vancouver (CA); Oleson, Mark Arthur, Portland (US); Hammond, Connor Alexander, Vancouver (CA); Ly, William, Vancouver (CA)
(74) Representative: Rommeswinkel, Marike

(56) References cited:
- CN-A- 103 445 787
- US-A1- 2017 202 271
- DEANA LORENTZEN ET AL: "Selected Sports Bras: A Biomechanical Analysis of Breast Motion While Jogging", PHYSICIAN AND SPORTSMEDICINE, MCGRAW-HILL, MINNEAPOLIS, US, vol. 15, no. 5, 1 January 1987 (1987-01-01), pages 128 - 139, XP009526656, ISSN: 0091-3847, Retrieved from the Internet <URL:http://www.tandfonline.com/doi/full/10.1080/00913847.1987.11709355> [retrieved on 20160711], DOI: 10.1080/00913847.1987.11709355
- MCGHEE DEIRDRE E ET AL: "Bra-breast forces generated in women with large breasts while standing and during treadmill running: Implications for sports bra design", APPLIED ERGONOMICS, vol. 44, no. 1, 27 June 2012 (2012-06-27), pages 112 - 118, XP028937998, ISSN: 0003-6870, DOI: 10.1016/J.APERGO.2012.05.006
- MONARI DAVIDE ET AL: "The effect of different breast support conditions on multiplanar breast kinematics and kinetics during running", GAIT & POSTURE, vol. 38, 21 November 2013 (2013-11-21), pages S95, XP028782095, ISSN: 0966-6362, DOI: 10.1016/J.GAITPOST.2013.07.196
- WOOD LOUISE ELLEN ET AL: "Predictors of Three-Dimensional Breast Kinematics during Bare-Breasted Running", MEDICINE AND SCIENCE IN SPORTS AND EXERCISE, vol. 44, no. 7, 1 July 2012 (2012-07-01), US, pages 1351 - 1357, XP055873332, ISSN: 0195-9131, DOI: 10.1249/MSS.0b013e31824bd62c
- ZHOU JIE ET AL: "Identifying effective design features of commercial sports bras", TEXTILE RESEARCH JOURNAL, vol. 83, no. 14, 27 November 2012 (2012-11-27), GB, pages 1500 - 1513, XP093305468, ISSN: 0040-5175, DOI: 10.1177/0040517512464289

## Description

### FIELD

This disclosure relates to a sensor and method for measuring movement of breast tissue during activity. In particular, the disclosure relates to a method for measuring acceleration of breast tissue during activity and a method of using acceleration measurements for recommending and fitting an undergarment.

### BACKGROUND

Typical athletic or sports bras are designed to restrict the movement of breast tissue related to activity by uniformly compressing the breast tissue to the wearer's chest. While the uniform compression effected by a typical athletic or sports bra may provide adequate movement management of the breast tissue, this compression can also be uncomfortable for the wearer because it does not effectively distribute the pressure around the wearer's torso. This discomfort is typically experienced around the wearer's back and shoulders. A typical athletic or sports bra completely captures and compresses the wearer's breast tissue to the wearer's chest, and is not designed to account for any specific directional movement or acceleration of the breast tissue resulting from the wearer's activity or the wearer's individual movement profile.

By failing to provide precise management of the breast tissue, a typical athletic or sports bra does not effectively maximize the balance between maintaining the comfort of the wearer and managing movement of the wearer's breast tissue.

Lorentzen, Deana, et al. Physician and Sports Medicine (1987), 15(5): 128-139 discloses a comparison between the design characteristics of different sports bras on vertical breast motion during jogging for women with different breast sizes. The range of biomechanical movement was calculated using the mean vertical displacement of the breast relative to the body during one average running stride.

McGhee, Deirdre, E., et al. Applied Ergonomics, (2013), 44: 112-118 disclose the use of vertical breast displacement and acceleration data to determine the bra-breast force generated during static upright standing and treadmill running while wearing bras with different support conditions.

CN103445787 discloses a chest model to evaluate the protective effect of bras on women's breasts. The model simulates the movement of breasts by detecting the movement tract of breasts before and after wearing a bra.

US2017/202271 discloses bras comprising a shape memory alloy, an accelerometer, a power source, a microprocessor and an algorithm implemented by the microprocessor, to learn and adapt to the displacement pattern of the wearer's breasts during physical activity.

There exists a need for measuring movement of breast tissue during activity, and then fitting an undergarment that provides more precise management of movement.

### SUMMARY

The present invention is defined by the appended claims.

In one aspect, the present disclosure provides a sensor for measuring movement or acceleration of breast tissue during activity and using the acceleration measurements in a method of fitting an undergarment for a user.

Various aspects of the present disclosure provide a sensor system (not claimed as such) for measuring movement of breast tissue during activity, the sensor system comprising: a first sensor for placement on a sternum of a user, the first sensor comprising a first accelerometer operable to generate acceleration data indicative of acceleration of a torso of the user along a reference x-axis, y-axis and/or z-axis; a second sensor operatively connected to the first sensor for placement on breast tissue of the user, the second sensor comprising a second accelerometer operable to generate acceleration data indicative of acceleration of breast tissue of the user; and a microprocessor operatively connected to the first and second sensors and configured to use the acceleration data generated from the first sensor and the second sensor to determine a magnitude of breast tissue acceleration relative to the torso of the user along the reference x-axis, y-axis and/or z-axis.

Various aspects of the present disclosure also provide a non-claimed method of fitting an undergarment for a user to control breast tissue acceleration during activity, the method comprising: receiving static breast parameters of the user, wherein the static breast parameters comprise one or more of a circumference of a torso of the user and a breast cup size of the user; measuring relative medial/lateral, anterior/posterior and/or superior/inferior breast tissue acceleration using a sensor as disclosed herein while the use is wearing a reference undergarment; comparing obtained data of the user's medial/lateral, anterior/posterior and/or superior/inferior breast tissue acceleration to a threshold level, wherein the threshold level is a range of combinations of at least two of medial/lateral, superior/inferior and anterior/posterior acceleration values that are perceived as comfortable or uncomfortable to a population of users for the reference undergarment at the static breast parameters; and selecting the undergarment from a collection of undergarments that controls the relative medial/lateral, anterior/posterior and/or superior/inferior breast tissue acceleration within the threshold level.

Various aspects of the present disclosure also provide a method of fitting an undergarment for a user to control breast tissue movement during activity, the method comprising: receiving static breast parameters of the user; determining an axial offset and/or a magnitude of breast tissue acceleration relative to the torso of the user along a reference x-axis, y-axis and/or z-axis; selecting, based on the static breast parameters and the axial offset and/or magnitude of breast tissue acceleration, an undergarment from among a collection of undergarments that controls the determined magnitude of breast tissue acceleration relative to the torso of the user within a threshold level, wherein the threshold level is a range of combinations of at least two of medial/lateral, superior/inferior and anterior/posterior acceleration values that are perceived as comfortable or uncomfortable to a population of users for the reference undergarment at the static breast parameters.

Various aspects of the present disclosure also provide a non-claimed method of generating a breast motion profile for a user, comprising: obtaining first acceleration data indicative of acceleration of a torso of the user along the reference x-axis, y-axis and/or z-axis; obtaining second acceleration data indicative of acceleration of breast tissue of the user; using the first and second acceleration data to determine an axial offset and/or a magnitude of breast tissue acceleration relative to the torso of the user; determining relative medial/lateral, anterior/posterior and/or superior/inferior breast tissue acceleration data from the determined magnitude of breast tissue acceleration relative to the torso of the user; comparing determined data of the user's medial/lateral, anterior/posterior and/or superior/inferior breast tissue acceleration to a database of known breast motion profiles to find a closest match; and generating the breast motion profile for the user by scaling the closest match based on the determined relative medial/lateral, anterior/posterior and/or superior/inferior breast tissue acceleration data.

Various aspects of the present disclosure also provide a computer-readable medium having stored thereon computer program code configured when executed by one or more processors to cause the one or more processors to perform a method as described herein.

Other aspects and features of the present disclosure will become apparent to those of ordinary skill in the art upon review of the following description of specific embodiments of the invention in conjunction with the accompanying claims.

### BRIEF DESCRIPTION OF THE DRAWINGS

In drawings which illustrate embodiments of the disclosure,
Figure 1 is a block diagram of a sensor system for measuring breast tissue acceleration.
Figure 2 is a plan view of a first housing for the first sensor
Figure 3 is a top view of a sensor system for measuring breast tissue acceleration
Figure 4 is a plan view of a second housing for the second sensor
Figure 5 is a diagram view of a sensor system for measuring breast tissue acceleration coupled to a user.
Figure 6 shows a user's breast displacement in the medial/lateral and superior/inferior axes as defining the two-dimensional view of the user's breast movement profile.
Figure 7 shows multiple users' medial/lateral and superior/inferior breast acceleration measurements compared to perceived comfort in order to determine a threshold level for an undergarment having specific static parameters.
Figure 8 shows a block diagram of a method of fitting an undergarment for a user to control breast tissue movement and/or acceleration during activity

### DETAILED DESCRIPTION

In the context of the present disclosure, various terms are used in accordance with what is understood to be the ordinary meaning of those terms.

Disclosed embodiments include systems, apparatus, methods and storage media associated with measuring breast tissue movement, including breast tissue acceleration. In various embodiments, the disclosure provides a sensor for measuring breast tissue acceleration and methods of using the acceleration data to fit an undergarment such as a bra for a user.

During activity, breast tissue has a range of motion in three dimensions, the medial/lateral (side-to-side) direction corresponding to movement along an x-axis, the superior/inferior (up and down) direction corresponding to movement along a y-axis and the anterior/posterior (in and out) direction corresponding to movement along a z-axis. Furthermore, this range of motion in three dimensions during activity is unique to each individual. In various embodiments, the disclosure provides a sensor and method for measuring acceleration of breast tissue in three dimensions relative to movement of a torso of a user. These measurements may then be used in a method of fitting an undergarment for a user. In various embodiments, the method uses the acceleration data to fit an undergarment, such as a bra, to control or decrease breast tissue acceleration in one or more of the medial/lateral, superior/inferior and/or anterior/posterior directions.

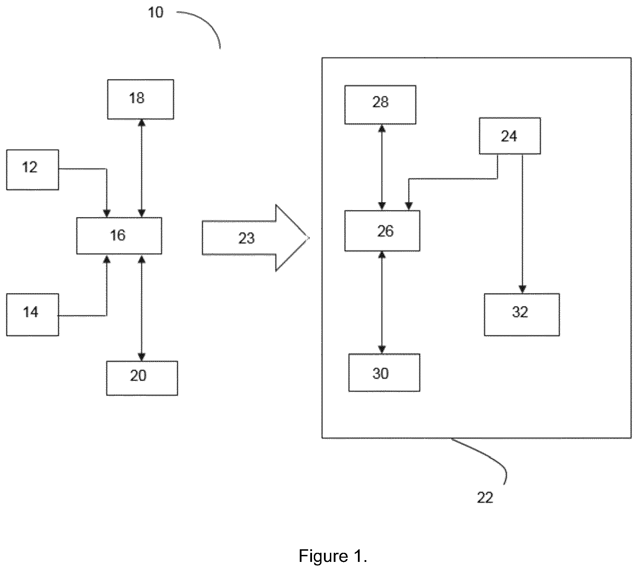

Referring to Figure 1 and according to a first embodiment of the disclosure, a sensor system **10** comprises a first sensor **12** and a second sensor **14.** The first sensor **12** is for placement on a sternum of the user and comprises a first accelerometer operable to generate data indicative of acceleration of a torso of the user. The first accelerometer is a triaxial accelerometer configured to generate data of torso acceleration along a reference x-axis, y-axis and z-axis. In various embodiments, the data indicative of acceleration of the torso of the user along the reference x-axis, y-axis and z-axis may serve as a reference vector.

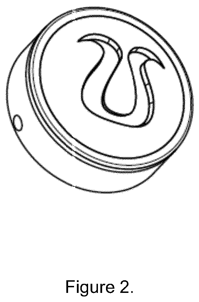

In various embodiments, the first sensor **12** is contained in a first housing. The first housing is designed to retain and protect the first accelerometer and other electronic components positioned within the first housing. The first housing may be of any shape, configuration or material that allows for coupling of the first sensor **12** to the sternum of the user. In various embodiments, the first housing includes a relatively rigid portion that securely retains the electronic components, and a more resilient portion which functions as an outer layer to provide shock absorption. The first sensor **12** may be provided as part of a chest strap or may include a clip or other arrangement that allows the first sensor **12** to be coupled to the sternum of the user. For example, the first housing may comprise a surface which faces the user and which can be attached to the user. In various embodiments, the surface of the first housing containing the first sensor **12** comprises an adhesive for placement on the sternum of the user. An example of a first housing **13** for the first sensor **12** is shown in Figure 2.

The first housing containing the first sensor **12** may also include other features visible on the first housing such as one or more coloured lights to communicate information, for example, the charge remaining in a battery.

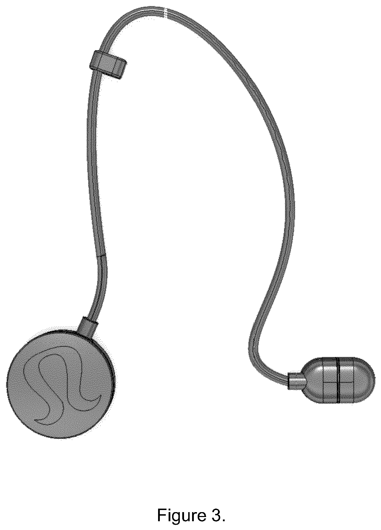

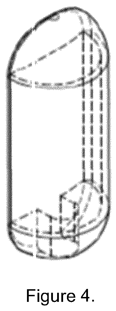

The second sensor **14** is operatively connected to the first sensor **12,** as shown, for example, in Figure 3. The second sensor **14** is for placement on breast tissue of the user. In various embodiments, the second sensor **14** is for placement over a nipple of the user. The second sensor **14** comprises a second accelerometer operable to generate acceleration data indicative of acceleration of breast tissue of the user. The second accelerometer is a second triaxial accelerometer. In various embodiments, the second accelerometer may generate breast tissue acceleration data in respect of one or more of any of the three dimensions (along an x-axis, a y-axis and/or a z-axis). The second sensor **14** is contained in a second housing that is designed to retain and protect the various components positioned within the second housing. The second housing may be of any shape, configuration or material that allows for positioning of the second sensor **14** over the breast tissue of the wearer. Figure 4 shows an example of the second housing **15** for the second sensor **14** according to an embodiment of the invention. In various embodiments, the second housing **15** includes a relatively rigid portion that securely retains the electronic components, and a more resilient portion which functions as an outer layer to provide shock absorption.

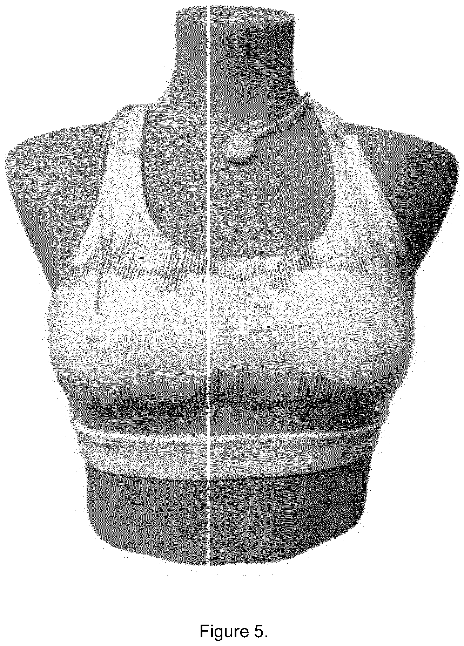

In various embodiments, the second sensor **14** may be positioned against the skin of the user, such as, for example, adhered to the skin of the user, or may be configured for placement in a receptacle on an article of clothing, such as a bra, over the breast tissue of the user as shown in Figure 5. In various embodiments, the receptacle on the article of clothing is on a surface of the article of clothing facing away from the user. Alternatively, the receptacle is on a surface of the article of clothing facing towards the user. In various embodiments, the second sensor **14** is placed in a receptacle on a bra that is worn by the user for purposes of measuring breast tissue acceleration. In various embodiments described herein, this bra may be referred to as a "reference bra".

In various embodiments, the orientation of each of the first sensor **12** and the second sensor **14** can affect the acceleration measurements, and therefore, the orientation of the sensors needs to be calibrated before positioning the first and second sensors **12** and **14** onto the body. In various embodiments, the orientation of the first and second sensors **12** and **14** needs to be within an upper and a lower threshold limit of the offset angle of the sensors **12** and **14** when positioned onto the body. For example, the rotation of the first and second sensors **12** and **14** needs to be within about ±10 degrees of the offset angle when positioned onto the body. Each of the first and second sensors **12** and **14** will detect the calibration values for each axis (x-, y-, and z-values) and convert them into an offset angle. If the offset angle is outside the threshold limits of about ±10 degrees, the sensor will send an output signal to the user to rotate the sensor in a pre-determined direction for the sensor to be properly aligned.

Once the sensor is oriented within the ±10 degrees threshold limits, it will send an output calibration signal to the user to attach the appropriate sensor onto the body. For example, once the first sensor **12** is oriented within the threshold limits, it will send a signal to the user to position the sensor on its pre-determined position onto the body, for example, onto the sternum. Once the second sensor **14** is oriented within the upper and lower threshold limits, it will send a signal to the user to position the sensor to its predetermined position onto the body, for example, onto the breast area.

The output calibration signal can be a visual output signal, such as a light or an image, a sound output signal, or a vibrational output signal. The processing of the calibration values for each of the first and second sensors **12** and **14** can be done by a sensor orientation calibration processor integrated within each of the sensors or that is separate from the first and second sensors **12** and **14** and is operatively connected to the sensors. The sensor orientation calibration processor will receive the calibration values for each of the sensors, process such values to calculate the offset angle of each sensor, compare if the offset angle is within the upper and lower threshold limits, and send an appropriate output signal to the user.

In various embodiments, the first and second triaxial accelerometers may be MEMS 14-bit, triaxial accelerometers. For example, the first and second triaxial accelerometers can measure three-dimensional acceleration from ground to ground, or stride to stride, contact of the user's feet during activity. For example, the activity may be any activity that involves a change of direction. In various embodiments, the activity may be running or any other activity such as, for example, walking, jumping, etc.

The sensor system **10** further comprises a microprocessor **16** operatively connected to the first and second sensors **12** and **14.** The microprocessor **16** can be the same or separate from the sensor orientation calibration processor. The microprocessor **16** may be any of various microprocessors as will be recognized by those of ordinary skill in the art. The microprocessor **16** is configured to receive data signals from the first and second sensors **12** and **14,** and other component parts of the sensor system **10,** and process such signals. For example, the first and second sensors **12** and **14** may generate two bits of data per stride of the user during activity which correspond to the peak to peak acceleration in m/s² for each stride in one or more of the x-axis, y-axis and z-axis. In various embodiments, the data from the first and second sensors **12** and **14** is synchronized to each stride of the user. In various embodiments, the microprocessor 16 is on the first sensor **12.** In other embodiments, the microprocessor 16 is on the second sensor **14.**

Raw acceleration data collected by the first and second sensors **12** and **14** may be processed by the microprocessor **16** and/or delivered to a remote server for further processing. Typical processing may include averaging the peak to peak measurements of acceleration along the reference x-axis, y-axis and/or z-axis from the first sensor **12** and/or averaging the peak to peak measurements of breast tissue acceleration from the second sensor **14** along each axis. For each axis, the sum of peak to peak acceleration may be divided by the number of data points (or strides).

Processing also includes using the acceleration data from the first and second sensors **12** and **14** to determine an axial offset and a magnitude of breast tissue acceleration relative to the acceleration of the torso of the user along the reference x-axis, y-axis and/or z-axis. Such processing may comprise comparing the average peak to peak acceleration of breast tissue along one axis to the average peak to peak acceleration of the torso along the same reference axis or vector. In various embodiments, the axial offset and magnitude of breast tissue acceleration may be processed to determine one or more of relative medial/lateral, anterior/posterior and/or superior/inferior breast tissue acceleration. Furthermore, the acceleration data may be processed into different forms and formats, depending on the particular device that will be ultimately used to view the data.

In various embodiments, the microprocessor **16** may use SPI to send data between the first or second sensors **12** and **14,** and the microprocessor **16.** The microprocessor **16** may be connected to a memory **18** and a first transceiver **20,** and may deliver processed data to one or both of the memory **18** and the first transceiver **20.** Additionally, the microprocessor **16** may perform processing of the received data prior to delivery thereof to the memory **18** or the first transceiver **20** as described above.

The memory **18** is configured to store information, including both data and instructions. The data generally include acceleration data that may be retrieved from the microprocessor **16** along with other data that may be ancillary to the basic operation of the microprocessor **16.** The instructions which are stored at the memory **18** generally include firmware and/or software for execution by the microprocessor **16,** such as a program that controls the settings for the first and second sensors **12** and **14,** a program that controls averaging peak to peak acceleration data from the first and second sensors **12** and **14,** a program that controls calibration of the orientation of the sensors **12** and **14,** a program that controls the processing of acceleration data from the first sensor **12** and the second sensor **14** to determine an axial offset and magnitude of breast tissue acceleration relative to the acceleration of the torso of the user along the reference x-axis, y-axis and z-axis, a program that controls the processing of acceleration data to determine relative medial/lateral, anterior/posterior and/or superior/inferior breast tissue acceleration with respect to the torso of the user, a program that controls the receipt of information via the first and second sensors **12** and **14,** a program that controls the transmission and reception of data via the first transceiver **20,** as well as any of various other programs that may be associated with the sensor system **10.** In various embodiments, two or more of the foregoing may be combined into one program.

The memory **18** may be of any type capable of storing information accessible by the microprocessor **16,** such as ROM, RAM, write-capable, read-only memories, or other computer-readable media. The data may also be formatted in any computer-readable format such as, but not limited to, binary values, ASCII or Unicode.

In various embodiments, the first transceiver **20** comprises a RF transmitter and receiver configured to transmit and receive communications signals over a short range using a wireless communications technology, such as Bluetooth^{®}, using any of various communications protocols. Such transceivers are well known to a person of ordinary skill in the art. The first transceiver **20** is configured to communicate with a display device **22** when the first transceiver **20** is within a given range of the display device **22,** and transmit acceleration data to the display device **22.** As represented by the arrow **23** in Figure 1, the first transceiver **20** is configured to transmit a wireless RF signal representative of acceleration data collected and obtained at the microprocessor **16** to at least the display device **22.**

The power source (not shown) is configured to provide power to one or more of the microprocessor **16** and the first and second sensors **12** and **14** during use. In various embodiments, the power source is a battery such as a rechargeable battery.

In various embodiments, the display device **22** may be a standalone device such as a desktop PC or smart television or any type of portable or other personal electronic device such as a smartphone, tablet computer, laptop computer, smartwatch, or any of various other mobile computing devices. As will be recognized by one of ordinary skill in the art, the components of the display device may vary depending on the type of display device used. The display device **22** generally includes an input/output interface **24,** a processor **26,** a memory **28** and a second transceiver **30.**

The I/O interface **24** of the display device **22** includes software and hardware configured to facilitate communications with the first transceiver **20** and/or communications to the user. The hardware includes a display screen **32** configured to visually display graphics, text and other data to the user. In particular, the display screen **32** is configured to display breast tissue movement or acceleration data received from the microprocessor **16** via the first transceiver **20.** The hardware may also include a microphone and/or speakers to facilitate audio communications with the user and/or verbal entry of commands to the device. In various embodiments, the display screen is a touch screen display that allows the user to see data presented on the display screen and input data into the display device via a keyboard on the touch screen.

The processor **26** of the display device **22** may be any of various processors as will be recognized by those of ordinary skill in the art. The processor **26** is connected to the I/O interface **24,** the memory **28,** and the second transceiver **30,** and is configured to deliver data to and/or receive data from each of these components. In various embodiments, the processor **26** is configured to process breast tissue movement or acceleration data received from the microprocessor **16** via the first transceiver **20** and transform the data into a graphical format for presentation on the display screen. As understood by a person of ordinary skill in the art, a "processor" as used herein includes any hardware system, hardware mechanism or hardware component that processes data, signals or other information. A processor can include a system with a central processing unit, multiple processing units, dedicated circuitry for achieving functionality, or other systems.

The memory **28** is configured to store information, including both data and instructions. The data may be breast tissue movement or acceleration data, along with other data that may be ancillary to the basic operation of the display device and any applications retained on the display device. The instructions which are stored at the memory **28** generally include firmware and other software for execution by the processor **26,** such as a program that controls the settings for the display device **22,** a program that controls the output of the display on the display device **22,** programs that control various applications on the display device **22,** a program that controls the transmission and reception of data via the second transceiver **30,** as well as any of various other programs that may be associated with the display device **22.** The instructions stored in the memory **28** for execution by the processor **26** may include, for example, an app for recommending an undergarment such as a bra to the user.

The memory **28** may be of any type of device capable of storing information accessible by the processor **26,** such as a memory card, ROM, RAM, write-capable memories, read-only memories, hard drives, discs, flash memory, or any of various other computer-readable medium serving as data storage devices as known by a person of ordinary skill in the art.

In at least one embodiment, portions of the system and methods described herein may be implemented in suitable software code that may reside within the memory (**18** and/or **28**). Such software code may be present on the device or microprocessor at the time of manufacture or may be downloaded thereto via well-known mechanisms. A computer program product implementing an embodiment disclosed herein may therefore comprise one or more computer-readable storage media storing computer instructions translatable by a processor or microprocessor to provide an embodiment of a system or perform an embodiment of a method disclosed herein. Computer instructions may be provided by lines of code in any of various languages as will be recognized by those of ordinary skill in the art. A "computer-readable medium" may be any type of data storage medium that can store computer instructions, including, but not limited to, the memory devices discussed above.

The second transceiver **30** is an RF transmitter and receiver configured to transmit and receive communications signals over a short range using wireless communications technology, such as Bluetooth^{®}, using any of various communications protocols. In various embodiments, the second transceiver **30** is configured to communicate with the first transceiver **20.**

The transmission of data from the first transceiver **20** to the display device **22** may occur automatically without the user needing to prompt the transmission. For example, some mechanism may be used to turn on the first transceiver **20** or otherwise indicate that automatic transmissions should begin. In another embodiment, the first transceiver **20** may be configured to begin transmissions once it receives a confirmation that the display device **22** is within an appropriate range of the first transceiver **20.** In yet another embodiment, data transmission may occur periodically at predetermined intervals of time.

The display device **22** also includes a battery or other power source (not shown) configured to power the various electronic components within the display device **22.**

In various embodiments, the memory (**18** and/or **28**) is configured to store data of a plurality of known breast motion profiles in a breast motion profile database. The axial offset and magnitude of breast tissue acceleration during an activity such as running, walking or jumping while wearing a particular bra or a reference bra can be measured using a number of motion capturing devices, and such data is processed to determine one or more of relative medial/lateral, anterior/posterior and/or superior/inferior breast tissue acceleration during the particular activity, and to determine a particular breast motion profile for the bra at certain static breast parameters, which is stored in the breast motion profile database. Computer instructions can also be provided by lines of code in any of various languages as will be recognized by those of ordinary skill in the art.

The data obtained from the first and the second sensors **12** and **14** are processed to determine the relative medial/lateral, anterior/posterior and/or superior/inferior breast tissue acceleration of the user wearing the particular bra or reference bra at specific static breast parameters during the particular activity and then the data of the relative medial/lateral, anterior/posterior and/or superior/inferior breast tissue acceleration is compared to the data included in the breast motion profile database in order to find the closest match which may be displayed to the user as a breast motion profile. In various embodiments, the data of the relative medial/lateral, anterior/posterior and/or superior/inferior breast tissue acceleration is scaled to determine the breast motion profile for display to the user.

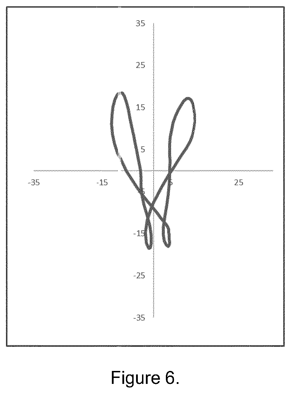

The database can also include data on a plurality of bras in addition to the reference bra which may form part of one or more bra recommendation areas or a selection of bras as discussed below, parameters relating to bras in the database which may include, for example, descriptions of "feeling" associated with each of the bras and differences in medial/lateral, anterior/posterior and/or superior/inferior acceleration measurements between the reference bra and each bra in the database, etc. For example, Figure 6 shows one format of a breast motion profile in which the personal breast tissue acceleration data are presented to the user via the screen **32.**

The acceleration data may be processed and displayed using the software application or "app" stored in a computer readable medium such as the memory **28** of the display device **22.** The processor of the display device is configured to process the instructions for the app. The processor **26** may be controlled by computer-executable instructions stored in memory **28** so as to provide functionality as is described herein. For example, the processor may process the breast tissue movement or acceleration data in order to present the data in a format for quickly and easily communicating the data to the user. The display device **22** includes a screen **32** configured to display the processed data.

In various embodiments, a non-transient computer readable medium contains instructions for controlling a display device by receiving breast tissue acceleration data from the microprocessor **16** and presenting a breast motion profile for the wearer on the display device and recommending an undergarment, such as a bra, to the wearer.

In various embodiments, the methods disclosed herein provide a customized fit and/or recommendation for undergarments, such as bras. The methods disclosed herein are based on an optimized fitting of the acceleration profile of breast tissue of the user with the functional characteristics of a bra to reduce or control that acceleration in each axis to a threshold level.

"Threshold level" refers to a range of combinations of at least two of medial/lateral, superior/inferior and anterior/posterior acceleration values that are perceived as comfortable or uncomfortable to a population of users for a particular bra or a reference bra at specific static breast parameters. The threshold level can be determined based on measurements of a population of users wearing a particular bra, such as the reference bra, for specific static breast parameters. In various embodiments, the threshold level may also depend on an activity being done by a user. For example, one undergarment may have a different threshold level for running as compared to the threshold level for yoga.

As used herein, the term "static breast parameters" refers to measureable characteristics of breast tissue when the user is not engaged in activity. Any value or combination of values below the threshold level for each of medial/lateral acceleration, superior/inferior acceleration and/or anterior/posterior acceleration may be viewed as providing support and comfort to a user during activity when wearing the particular bra.

Depending on a user's breast motion profile, the user may require an undergarment which provides for greater reduction in medial/lateral acceleration in order to be comfortable during activity, or the user may require an undergarment which provides for greater reduction in anterior/superior acceleration in order to feel comfortable during activity. Alternatively, the user may require an undergarment which provides for approximately equal reduction in both medial/lateral and anterior/superior acceleration in order to feel comfortable during activity.

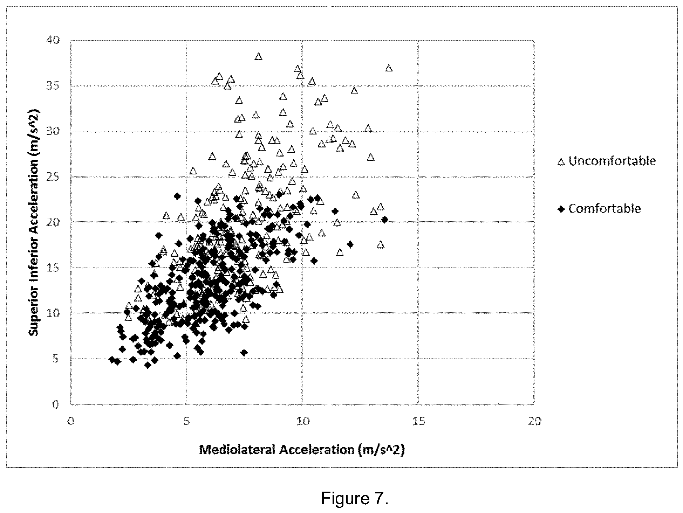

As shown in Figure 7, various combinations of values for medial/lateral and superior/inferior acceleration may be perceived as comfortable or uncomfortable by a population of users when wearing a particular bra or a reference bra for specific static breast parameters and therefore defines the threshold level. The methods as disclosed herein recommend an undergarment to the user that decreases one or more of the medial/lateral acceleration, superior/inferior acceleration or anterior/posterior acceleration so that the user is more comfortable compared to the reference bra.

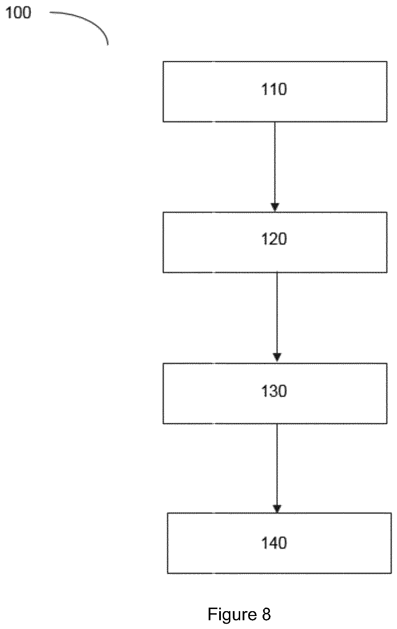

An exemplary embodiment of the methods disclosed herein is shown in Figure 8. In various embodiments of a method (100) for fitting an undergarment for a user to control or reduce breast tissue movement or acceleration during an activity, the method includes providing input on static breast parameters of the user (110), the static breast parameters may comprise a circumference of a torso of the user and a breast cup size of the user. For example, user underband and bust size can be measured to determine bra size and a size of a reference bra that the user can use during an activity. The static breast parameters may be input to the display device **22.**

The method further includes measuring breast tissue acceleration (120) using a sensor system as described herein. For example, the breast tissue acceleration of the user may be measured while the user wears the reference bra during the activity, such as running. Data obtained from the sensors **12** and **14** are processed to obtained the user's medial/lateral and superior/inferior breast tissue acceleration (peak to peak) and compare the value of the user's breast acceleration to the acceleration data in the breast motion profile database to find the closest match and determine the user's breast movement profile.

The user's breast tissue acceleration data is compared to the threshold level for the reference bra at the inputted static breast parameters (130). For example, the measured medial/lateral and/or superior/inferior acceleration may have higher values than the threshold level of the reference bra at the inputted static breast parameters and the user may experience discomfort while undertaking the activity while wearing the reference bra. In this example, the measured axial offset and/or magnitude of breast tissue acceleration exceeds the threshold level.

The processor **26** processes the data related to the breast acceleration profile and provides an output or a recommendation to the user for an undergarment (i.e., bra) in the user's size so that the breast acceleration remains below the threshold level for the recommended undergarment. Thus, the recommended undergarment provides the desired control for the user's unique breast motion profile in order to increase the user's comfort while engaged in the activity (140).

For example, if the user's acceleration profile indicates greater values of acceleration in the medial/lateral direction compared to the threshold level for the reference bra, then the method recommends a bra from a selection of bras (also referred to as the "bra recommendation area") that provides more control in the medial/lateral direction compared to the reference bra. If the user's acceleration profile indicates greater values of acceleration in both the superior/inferior and the medial/lateral directions compared to the threshold level for the reference bra, then the method recommends a bra from a selection of bras or from a bra recommendation area that provides more control in the superior/inferior and medial/lateral directions compared to the reference bra.

In various embodiments, the extent to which each bra in the selection of bras or bra recommendation area decreases one or more of the medial/lateral, superior/inferior and/or anterior/posterior acceleration is pre-determined based on the obtained data of the acceleration profile and this data is stored in the display device **22.** Wearing the recommended bra, the user experiences an increased level of comfort, as a result of reduced medial/lateral, superior/inferior, and/or anterior/posterior acceleration of breast tissue during activity.

In various embodiments, the method may further comprise receiving comfort perception data from the user. Such comfort perception data may include intended activity of the user while wearing the undergarment and other characteristics preferred by a user. The higher the intensity of intended activity of the user while wearing the undergarment, generally the greater the reduction in breast tissue acceleration required by the user. "Perception" as used herein refers to a personal feeling or subjective perception of one's own feeling of comfort as determined by the user based on the user's own senses, feelings, awareness, mental impressions or other perceptions of the user.

Although various embodiments of the invention are disclosed herein, many adaptations and modifications may be made within the scope of the invention, which is defined by the appended claims, in accordance with the common general knowledge of those skilled in this art. Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing. Citation of references herein is not an admission that such references are prior art to the present invention.

## Claims

1. A method (100) of fitting an undergarment for a user to control breast tissue movement during activity, the method comprising:
receiving static breast parameters of the user (110);
determining an axial offset and/or a magnitude of breast tissue acceleration relative to the torso of the user along a reference x-axis, y-axis and/or z-axis while the user is wearing a reference undergarment (120) using a sensor system (10) comprising a first sensor (12) for placement on a sternum of the user, the first sensor (12) comprising a first accelerometer operable to generate acceleration data indicative of acceleration of the torso of the user along the reference x-axis, y-axis and/or z-axis, a second sensor (14) operatively connected to the first sensor (12) for placement on breast tissue of the user, the second sensor (14) comprising a second accelerometer operable to generate acceleration data indicative of acceleration of breast tissue of the user and a microprocessor operatively connected to the first and second sensors (12, 14) and configured to use the acceleration data from the first sensor (12) and the second sensor (14) to determine the axial offset and/or the magnitude of breast tissue acceleration relative to the torso of the user along the reference x-axis, y-axis and/or z-axis; and
selecting, based on the static breast parameters and the axial offset and/or magnitude of breast tissue acceleration, an undergarment from among a collection of undergarments that controls the determined magnitude of breast tissue acceleration relative to the torso of the user within a threshold level (130), wherein the threshold level is a range of combinations of at least two of medial/lateral, superior/inferior and anterior/posterior acceleration values for the reference undergarment at the static breast parameters.

2. The method of claim 1, wherein the static breast parameters comprise one or more of a circumference of a torso of the user and a breast cup size of the user.

3. The method of claim 1 or 2, further comprising determining relative medial/lateral, anterior/posterior and/or superior/inferior breast tissue acceleration data from the determined magnitude of breast tissue acceleration relative to the torso of the user, and wherein selecting the undergarment (130) comprises selecting, based on the medial/lateral, anterior/posterior and/or superior/inferior breast tissue acceleration data, an undergarment from among the collection of undergarments that controls a magnitude of medial/lateral, anterior/posterior and/or superior/inferior breast tissue acceleration within the threshold level.

4. The method of any preceding claim, further comprising receiving comfort perception data from the user.

5. The method of claim 4, wherein the comfort perception data comprises intended activity of the user while wearing the undergarment.

6. A computer-readable medium having stored thereon computer program code configured when executed by one or more processors to cause the one or more processors to perform a method as defined in any one of claims 1 to 5.

## Patentansprüche

1. Verfahren (100) zum Anpassen eines Unterwäschestücks an eine Benutzerin, um die Bewegung des Brustgewebes während einer Aktivität zu kontrollieren, wobei das Verfahren umfasst:
Empfangen statischer Brustparameter der Benutzerin (110);
Bestimmen eines axialen Versatzes und/oder einer Größe der Beschleunigung des Brustgewebes relativ zum Oberkörper der Benutzerin entlang einer Referenz-x-Achse, y-Achse und/oder z-Achse, während die Benutzerin ein Referenzunterwäschestück (120) trägt, unter Verwendung eines Sensorsystems (10), das einen ersten Sensor (12) zum Anbringen am Brustbein der Benutzerin umfasst, wobei der erste Sensor (12) einen ersten Beschleunigungsmesser umfasst, der Beschleunigungsdaten erzeugen kann, die die Beschleunigung des Oberkörpers der Benutzerin entlang der Referenz-x-Achse, y-Achse und/oder z-Achse anzeigen, einen zweiten Sensor (14), der funktionsfähig mit dem ersten Sensor (12) verbunden ist, um auf dem Brustgewebe der Benutzerin platziert zu werden, wobei der zweite Sensor (14) einen zweiten Beschleunigungsmesser umfasst, der betätigt werden kann, um Beschleunigungsdaten zu erzeugen, die die Beschleunigung des Brustgewebes der Benutzerin anzeigen, und einen Mikroprozessor, der funktionsfähig mit dem ersten und dem zweiten Sensor (12, 14) funktionsfähig verbunden ist und so konfiguriert ist, dass er die Beschleunigungsdaten vom ersten Sensor (12) und vom zweiten Sensor (14) verwendet, um den axialen Versatz und/oder die Größe der Beschleunigung des Brustgewebes relativ zum Oberkörper der Benutzerin entlang der Referenz-x-Achse, y-Achse und/oder z-Achse zu bestimmen; und
Auswählen, basierend auf den statischen Brustparametern und dem axialen Versatz und/oder der Größe der Brustgewebebeschleunigung, eines Unterwäschestücks aus einer Sammlung von Unterwäschestücken, das die bestimmte Größe der Brustgewebebeschleunigung relativ zum Oberkörper der Benutzerin innerhalb eines Schwellenwertbereichs (130) kontrolliert, wobei der Schwellenwert ein Bereich von Kombinationen aus mindestens zwei der medialen/lateralen, superioren/inferioren und anterioren/posterioren Beschleunigungswerte für die Referenzunterwäsche bei den statischen Brustparametern ist.

2. Verfahren nach Anspruch 1, wobei die statischen Brustparameter einen oder mehrere der folgenden Werte umfassen: einen Umfang des Oberkörpers der Benutzerin und eine Körbchengröße der Benutzerin.

3. Verfahren nach Anspruch 1 oder 2, das ferner das Bestimmen relativer medialer/lateraler, anteriorer/posteriorer und/oder superiorer/inferiorer BrustgewebeBeschleunigungsdaten aus der bestimmten Größe der Brustgewebe-Beschleunigung relativ zum Oberkörper der Benutzerin umfasst, und wobei das Auswählen der Unterwäsche (130) das Auswählen auf der Grundlage der medialen/lateralen, anterioren/posterioren und/oder superioren/inferioren Brustgewebebeschleunigungsdaten eine Unterwäsche aus der Unterwäschekollektion auszuwählen, die eine Größe der medialen/lateralen, anterioren/posterioren und/oder superioren/inferioren Brustgewebebeschleunigung innerhalb des Schwellenwertbereichs steuert.

4. Das Verfahren nach einem der vorstehenden Ansprüche, das ferner das Empfangen von Komfortwahrnehmungsdaten von der Benutzerin umfasst.

5. Verfahren nach Anspruch 4, wobei die Komfortwahrnehmungsdaten die beabsichtigte Aktivität der Benutzerin während des Tragens der Unterwäsche umfassen.

6. Ein computerlesbares Medium, auf dem ein Computerprogrammcode gespeichert ist, der so konfiguriert ist, dass er bei Ausführung durch einen oder mehrere Prozessoren bewirkt, dass der eine oder die mehreren Prozessoren ein Verfahren gemäß einem der Ansprüche 1 bis 5 ausführen.

## Revendications

1. Procédé (100) d'ajustement d'un sous-vêtement pour une utilisatrice afin de contrôler le mouvement du tissu mammaire pendant l'activité, le procédé comprenant:
la réception des paramètres statiques des seins de l'utilisatrice (110);
déterminer un décalage axial et/ou une amplitude d'accélération du tissu mammaire par rapport au torse de l'utilisateur le long d'un axe x de référence, l'axe y et/ou l'axe z pendant que l'utilisateur porte un sous-vêtement de référence (120) à l'aide d'un système de capteurs (10) comprenant un premier capteur (12) destiné à être placé sur le sternum de l'utilisateur, le premier capteur (12) comprenant un premier accéléromètre pouvant fonctionner pour générer des données d'accélération indiquant l'accélération du torse de l'utilisateur le long de l'axe x de référence, l'axe y et/ou l'axe z, un deuxième capteur (14) connecté de manière opérationnelle au premier capteur (12) pour être placé sur le tissu mammaire de l'utilisateur, le deuxième capteur (14) comprenant un deuxième accéléromètre pouvant fonctionner pour générer des données d'accélération indiquant l'accélération du tissu mammaire de l'utilisateur et un microprocesseur connecté de manière opérationnelle aux premier et deuxième capteurs (12, 14) et configuré pour utiliser les données d'accélération provenant du premier capteur (12) et du deuxième capteur (14) afin de déterminer le décalage axial et/ou l'amplitude de l'accélération du tissu mammaire par rapport au torse de l'utilisatrice le long de l'axe x, de l'axe y et/ou de l'axe z de référence; et
sélectionner, sur la base des paramètres statiques des seins et du décalage axial et/ou de l'amplitude de l'accélération du tissu mammaire, un sous-vêtement parmi une collection de sous-vêtements qui contrôle l'amplitude déterminée de l'accélération du tissu mammaire par rapport au torse de l'utilisatrice dans une plage seuil (130), dans lequel le niveau seuil est une plage de combinaisons d'au moins deux des valeurs d'accélération médiale/latérale, supérieure/inférieure et antérieure/postérieure pour le sous-vêtement de référence aux paramètres mammaires statiques.

2. Procédé selon la revendication 1, dans lequel les paramètres statiques des seins comprennent un ou plusieurs des éléments suivants: la circonférence du torse de l'utilisatrice et la taille du bonnet de soutien-gorge de l'utilisatrice.

3. Procédé selon la revendication 1 ou 2, comprenant en outre la détermination de données d'accélération relative du tissu mammaire médial/latéral, antérieur/postérieur et/ou supérieur/inférieur à partir de l'amplitude déterminée de l'accélération du tissu mammaire par rapport au torse de l'utilisatrice, et dans lequel la sélection du sous-vêtement (130) comprend la sélection, sur la base des données d'accélération du tissu mammaire médial/latéral, antérieure/postérieure et/ou supérieure/inférieure du tissu mammaire, un sous-vêtement parmi la collection de sous-vêtements qui contrôle une amplitude d'accélération médiale/latérale, antérieure/postérieure et/ou supérieure/inférieure du tissu mammaire dans la limite du seuil.

4. Le procédé selon l'une quelconque des revendications précédentes, comprenant en outre la réception de données de perception du confort provenant de l'utilisateur.

5. Procédé selon la revendication 4, dans lequel les données de perception du confort comprennent l'activité prévue de l'utilisateur pendant le port du sous-vêtement.

6. Support lisible par ordinateur sur lequel est stocké un code de programme informatique configuré pour, lorsqu'il est exécuté par un ou plusieurs processeurs, amener le ou les processeurs à exécuter un procédé tel que défini dans l'une quelconque des revendications 1 à 5.
